Europäisches Patentamt

European Patent Office

Office européen des brevets

(11) Publication number: **0 136 489**
**B1**

# EUROPEAN PATENT SPECIFICATION

(45) Date of publication of patent specification: **25.11.87**

(21) Application number: **84109537.5**

(22) Date of filing: **10.08.84**

(51) Int. Cl.⁴: **C 12 N 15/00, C 12 P 21/02, C 07 H 21/04**

(54) Analogs of human interleukin II and their preparation.

(30) Priority: 10.08.83 US 521967
03.08.84 US 635941

(43) Date of publication of application:
10.04.85 Bulletin 85/15

(45) Publication of the grant of the patent:
25.11.87 Bulletin 87/48

(84) Designated Contracting States:
AT BE CH DE FR GB IT LI LU NL SE

(56) References cited:
EP-A-0 088 195
EP-A-0 091 539
EP-A-0 109 748
EP-A-0 119 621

NATURE, vol. 302, 24th March 1983, pages 305-310, Macmillan Journals Ltd.; T.

The file contains technical information submitted after the application was filed and not included in this specification

(73) Proprietor: **Amgen**
**1900 Oak Terrace Lane**
**Thousand Oaks California 91320 (US)**

(72) Inventor: **Souza, Lawrence M.**
**1302 Witherspoon**
**Thousand Oaks California 91360 (US)**
Inventor: **Stabinsky, Yitzhak**
**19 Horwitz Street**
**Rishon Le-Zion (IL)**

(74) Representative: **Brown, John David et al**
**FORRESTER & BOEHMERT**
**Widenmayerstrasse 4/I**
**D-8000 München 22 (DE)**

(56) References cited:

TANIGUCHI et al.: "Structure and expression of a cloned cDNA for human interleukin-2"

NUCLEIC ACIDS RESEARCH, vol. 11, no. 13, July 1983, pages 4307-4323, IRL Press Ltd., Oxford, GB; R. DEVOS et al.: "Molecular cloning of human interleukin 2 cDNA and its expression in E. coli"

## Description

### BACKGROUND

The present invention relates to polypeptide analogs of Interleukin II, compositions containing such analogs and processes of preparing such analogs.

Interleukin II ("IL—II"), a glycoprotein with a molecular weight of approximately 15,000, is a member of a group of proteins, called lymphokines, that control the body's immune response. IL—II is produced by certain white blood cells, lectin- or antigen-activated T cells, and plays a central role in the body's immune system as a lymphocyte regulating molecule.

At one time generally referred to as T-cell growth factor, IL—II has been repoted to enhance thymocyte mytogenesis, to stimulate long-term *in vitro* growth of activated T-cell clones, to induce cytotoxic T-cell reactivity, to induce plaque-forming cell responses in cultures of nude mouse spleen cells, and to regulate production of gamma interferon. It also augments natural killer cell activity and mediates the recovery of the immune function of lymphocytes in selected immunodeficient states.

Additionally, in the laboratory, it could be used to maintain cultures of functional monoclonal T-cells to study the molecular nature of T-cell differentiation, and to help elicit the mechanism of differentiated T-cell functions.

Thus, IL—II has potential application in both research and the treatment of neoplastic and immunodeficiency diseases. However, the limited amount of purified native IL—II obtainable from peripheral blood lymphocytes and tumor cell lines has been an impediment to studies of the biological role of this lymphokine.

Taniguchi, T., et al., *Nature, 302:* 305—310 (1983) described the sequence analysis, cloning, and expression of a complementary DNA coding for human IL—II, cloned from a cDNA library prepared from partially purified IL—II mRNA from the Jurkat leukemia cell line. IL—II was proposed to comprise 133 amino acid residues and to have a calculated molecular weight of about 15,420. Taniguchi described the cloning procedures and the expression of the cDNA for IL—II in cultured monkey COS cells. The publication states that expression of the IL—LL cDNA in *E. coli* which is necessary to produce large quantities of the protein had not yet been accomplished.

Devos, R *et al.,* Nucl. Acids Research, 11; 4307—4323 have also reported the molecular cloning of human IL—II cDNA and its expression in E. coli. The deduced aminoacid sequence for IL—II reported by these authors is in complete agreement with that given by Taniguchi *et al.* (loc. cit.).

More recently, Rosenberg, et al., *Science, 223:* 1412—1415 (1984) reported the isolation of another cDNA clone of the IL—II gene from the Jurkat tumor cell line and from normal human peripheral blood lymphocytes. These researchers inserted the gene into *E. coli,* purified the polypeptide product and assayed it for biological activity.

Considerable interest therefore exists in the development of methods and materials for the production of large amounts of purified IL—II to replace, crude, IL—II-containing lymphokine preparations currently employed in immunotherapy research and further study of the biological role of this protein in immunologic processes.

### BRIEF SUMMARY

There is disclosed herein manufactured genes capable of directing synthesis, in selected microbial hosts (e.g., bacteria, yeast and mammalian cells in culture), of human Interleukin II ("IL—II"). In preferred forms of manufactured genes, the base sequence includes one or more codons selected from among alternative codons specifying the same amino acid on the basis of preferential expression characteristics for the coden in a projected host microorganism, e.g., *E. coli.*

Other preferred forms of manufactured genes include those wherein there is provided a base codon specifying an additional amino acid residue in the polypeptide coded for which facilitates the direct expression in *E. coli* organisms (e.g., an initial Met residue). In still other preferred forms of manufactured genes, the sequence of base codons specifying the desired polypeptide is preceded by and/or followed by and/or includes one or more sequences of bases facilitating formation of expression vectors or generation of new structural genes for polypeptide analogs, i.e., sequences of bases providing for selected restriction endonuclease cleavage sites on one or both ends of the structural gene or at intermediate positions therein.

Also discussed herein are manufactured genes capable of directing the microbial expression of human Interleukin II polypeptide analogs wherein one of the three cysteine residues of the naturally occurring form of the polypeptide is replaced by another aminoacid residue, such as serine or alamine.

According to the first aspect of the present invention there is provided a polypeptide analog of human Interleukin II having the cysteine residue at amino acid position 125 replaced, whilst substantially retaining the biological activity of the natural form.

According to the second aspect of the present invention there is provided à composition containing a polypeptide analog in accordance with the first aspect of the present invention.

According to a third aspect of the present invention there is provided a process of preparing a polypeptide analog of human Interleukin II which process comprises:

a) manufacturing a synthetic gene capable of disecting the synthesis in a selected host microorganism of a human Interleukin II polypeptide;

0 136 489

b) altering in said gene the codon specifying the cysteine residue in position 125 so as to replace it with a codon specifying another amino acid residue;

c) transforming a selected host microorganism with a biologically functional DNA including said altered synthetic gene of b) and growing, under appropriate nutrient conditions, said transformed microorganism, whereby said microorganism expresses said gene and produces the polypeptide analog.

To generate polypeptide products, manufactured DNA sequences are interested into viral or circular plasmid DNA vectors to form hybrid vectors and the hybrid vectors are employed to transform microbial hosts such as bacteria (e.g., *E. coli*), yeast cells, or mammalian cells in culture. The transformed microorganisms are thereafter grown under appropriate nutrient conditions and express the polypeptide products of the invention.

Other aspects and advantages of the present invention will be apparent upon consideration of the following detailed description thereof.

DETAILED DESCRIPTION

As employed herein, the term "manufactured" as applied to a DNA sequence or gene shall designate a product either totally chemically synthesized by assembly of nucleotide bases or derived from the biological replication of a product thus chemically synthesized. As such, the term is exclusive of products "synthesized" by cDNA methods or genomic cloning methodologies which involve starting matrials which are initially of biological origin.

The following abbreviations shall be employed herein to designate amino acids: Alanine, Ala; Arginine, Arg; Asparagine, Asn; Aspartic acid, Asp; Cysteine, Cys; Glutamine, Gln; Glutamic acid, Glu; Glycine, Gly; Histidine, His; Isoleucine, Ile; Leucine, Leu; Lysine, Lys; Methionine, Met; Phenylalanine, Phe; Proline, Pro; Serine, Ser; Threonine, Thr; Tryptophan, Trp; Tyrosine, Tyr; Valine, Val. The following abbreviations shall be employed for nucleotide bases: A for adenine; G for guanine; T for thymine; U for uracil; and C for cytosine.

For easy of understanding of the present invention, Table I below provides a tabular correlation between the 64 alternate triplet nucleotide base codons of DNA and the 20 amino acids and transcription termination ("stop") function specified thereby.

## TABLE I

| FIRST POSITION | SECOND POSITION | | | | THIRD POSITION |
|---|---|---|---|---|---|
| | T | C | A | G | |
| T | Phe | Ser | Tyr | Cys | T |
| | Phe | Ser | Tyr | Cys | C |
| | Leu | Ser | Stop | Stop | A |
| | Leu | Ser | Stop | Trp | G |
| C | Leu | Pro | His | Arg | T |
| | Leu | Pro | His | Arg | C |
| | Leu | Pro | Gln | Arg | A |
| | Leu | Pro | Gln | Arg | G |
| A | Ile | Thr | Asn | Ser | T |
| | Ile | Thr | Asn | Ser | C |
| | Ile | Thr | Lys | Arg | A |
| | Met | Thr | Lys | Arg | G |
| G | Val | Ala | Asp | Gly | T |
| | Val | Ala | Asp | Gly | C |
| | Val | Ala | Glu | Gly | A |
| | Val | Ala | Glu | Gly | G |

3

The manufacture of structural genes is preferably carried out according to the procedures disclosed in the co-owned, co-pending U.S. Patent Application Serial No. 375,493, filed May 6, 1982 [PCT International Publication No. WO83/04029, published November 24, 1983] by Yitzhak Stabinsky entitled "Manufacture and Expression of Structural Genes", which application is incorporated by reference herein for the purpose of describing the steps of the procedure.

Briefly summarized, the general method comprises the steps of:

(1) preparing two or more different, linear, duplex DNA strands, each duplex strand including a double-stranded region of 12 or more selected complementary base pairs and further including a top single-stranded terminal sequence of from 3 to 7 selected bases at one end of the strand and/or a bottom single-stranded terminal sequence of from 3 to 7 selected bases at the other end of the strand, each single-stranded terminal sequence of each duplex DNA strand comprising the entire base complement of at most one single-stranded terminal sequence of any other duplex DNA strand prepared; and

(2) annealing and ligating each duplex DNA strand prepared in step (1) to one or two different duplex strands prepared in step (1) having a complementary single-stranded terminal sequence, thereby to form a single continuous double-stranded DNA sequence which has a duplex region of at least 27 selected base pairs including at least 3 base pairs forms by complementary association of single-standed terminal sequences of duplex DNA strands prepared in step (1) and which has from zero to 2 single-stranded top or bottom terminal regions of from 3 to 7 bases.

In the preferred general process for subunit manufacture, at least three different duplex DNA strands are prepared in step (1) and all strands so prepared are annealed and ligated concurrently in a single reaction mixture to form a single continuous double-stranded DNA sequence which has a duplex region of at least 42 selected base pairs including at least two non-adjacent sets of 3 or more base pairs formed by complementary association of single-stranded terminal sequences of duplex strands prepared in step (1).

The duplex DNA strand preparation step (1) of the preferred subunit manufacturing processing preferably comprises the steps of:

(a) constructing first and second linear deoxyoligonucleotide segments having 15 or more bases in a selected linear sequence, the linear sequence of bases of the second segment comprising the total complement of the sequence of bases of the first segment except that at least one end of the second segment shall either include an additional linear sequence of from 3 to 7 selected based beyond those fully complementing the first segment, or shall lack a linear sequence of from 3 to 7 bases complementary to a terminal sequence of the first segment, provided, however, that the second segment shall not have an additional sequence of bases or be lacking a sequence of bases at both of its ends; and

(b) combining the first and second segments under conditions conducive to complementary association between segments to form a linear, duplex DNA strand.

The sequence of bases in the double-stranded DNA subunit sequences formed preferably includes one or more triplet codons selected from among alternative codons specifying the same amino acid on the basis of preferential expression characteristics of the codon in a projected host microorganism, such as yeast cells or bacteria, especially *E. coli* bacteria.

The following examples illustrate in the manufacture of the DNA sequences coding for microbial expression of human IL—II and polypeptide analogs thereof. Also illustrated is the construction of expression vectors for microbial expression of desired polypeptides.

The following example is directed to the general procedure employed in the synthesis of oligonucleotide sequences employed to manufacture structural genes.

## Example 1

Oligonucletotide sequences were synthesized using a four-step procedure and several intermediate washes. Polymer bound dimethoxytrityl protected nucleoside in a sintered glass funnel was first stripped of its 5'-protecting group (dimethoxytrityl) using 2% trichloroacetic acid in dichloromethane for 1-1/2 minutes. The polymer was then washed with methanol, tetrahydrofuran and acetonitrile. The washed polymer was then rinsed with dry acetonitrile, placed under argon and then treated in the condensation step as follows. 0.6 ml of a solution of 10 mg tetrazole in acetonitrile was added to the reaction vessel containing polymer. Then 0.4 ml of 30 mg protected nucleoside phosphoramidite in acetonitrile was added. This reaction was agitated and allowed to react for 2 minutes. The reactants were then removed by suction. and the polymer rinsed with acetonitrile. This was followed by capping the unreacted 5'-hydroxyl groups using a solution prepared by mixing one part acetic anhydride in 2,6-lutidine (v/v) and four parts of 0.6M dimethylaminopyridine in tetrahydrofuran. After two minutes the capping solution was sucked and the polymer was treated for two minutes with an oxidizing solution (0.1M $I_2$ in 2,6-lutidine/$H_2O$/THF, 1:2:2). This was followed by an acetonitrile and $CH_2Cl_2$ rinse. The cycle began again with a trichloroacetic acid in $CH_2Cl_2$ treatment and was repeated until the desired oligonucleotide sequence was obtained.

The final oligonucleotide chain was treated with thiophenol dioxane, triethylamine 1:2:2, for 75 minutes at room temperature. Then, after rinsing with dioxane, methanol, and diethylether, the oligonucleotide was cleaved from the polymer with concentrated ammonia at room temperature. After decanting the solution from the polymer, the concentrated ammonia solution was heated at 60°C for 16 hours in a sealed tube.

Each oligonucleotide solution was then extracted once with 1-butanol. The solution was loaded into a

4

**0 136 489**

15% polyacrylamide 7 molar urea.electrophoresis gel and, after running, the appropriate product band was isolated.

The following example is directed to the manufacture of structural genes coding for human IL—II and also illustrates the manner in which structural genes for polypeptide analogs thereof may be prepared.

Example 2

A structural gene coding for human IL—II was manufactured in two parts. Fragment No. 1 of IL—II included codons specifying the initial 34 complete codons of the amino terminal of the polypeptide, a translation initiation coden sequence (specifying Met$^{-1}$ residue) and a desirable ribosome binding site sequence of bases 5' to the protein coding region. In the manufacture of Fragment No. 1 of the IL—II gene, ten specific deoxyoligonucleotides (numbered 1 through 10) were synthesized according to the procedures of Example 1. The oligonucleotide sequences were purified by polyacrylamide gel electrophoresis and were phosphorylated at the 5' ends using ATP and T—4 polynucleotide kinase in a standard reaction using 0.5 nanomole of DNA, a two fold excess of ATP and 1 unit of T—4 kinase in 25 µl of buffer made with 50 mM hydroxyethylpiperazine ethane sulfonic acid, 10 mM $MgCl_2$, 10 mM dithiothrietol, pH 7.6. After reaction, the kinase was destroyed by boiling for 5 minutes. These phosphorylated oligonucleotides in the buffer were then used directly for ligation.

The oligonucleotides in 25 µl standard buffer were combined to form short duplexes. Each duplex was formed by combining two complementary sequences in equimolar amounts, boiling the mixture, then slow cooling over a 1/2 hour period to room temperature. In this way, five duplexes were formed (oligonucleotides 1 and 2 forming duplex I, oligonucleotides 3 and 4 forming duplex II, and so on).

Table II below illustrates the finally assembled Fragment No. 1 for a gene coding for microbial expression of human IL—II. In the course of assembly of Fragment No. 1, duplexes I and II were combined in one tube while duplexes III, IV, and V were combined in a second. The ATP concentrations in each tube were adjusted to be 250 µM and T—4 DNA ligase (5 units per nanomole of deoxyoligonucleotide). After one hour the two mixtures were combined and the ligation reaction was allowed to proceed for another six hours at room temperature. The 134 base pair product was then purified by polyacrylamide gel electrophoresis.

## TABLE II

### IL-II FRAGMENT NO. 1

__Xba I__

```
                    I                                      II
      ┌──────────1─────────┐                  ┌────────3────────
CTAGAAAAAA  CCATGAGGGT  AATAAATAAT  GGCTCCTACG  AGCTCTTCTA
    TTTTTT  GGTACTCCCA  TTATTTATTA  CCGAGGATGC  TCGAGAAGAT
      └─────────2──────────┘                  └────────4────────


                   III                                    IV
      ┌──────────5─────────┐                  ┌────────7────────
CTAAGAAAAC  CCAGCTGCAA  CTGGAACATC  TGCTTCTTGA  CCTGCAAATG
GATTCTTTTG  GGTCGACGTT  GACCTTGTAG  ACGAAGAACT  GGACGTTTAC
      └──────────6─────────┘                  └────────8────────


                    V
      ┌──────────9───────────────────────┐
ATCCTGAACG  GTATCAACAA  CTACAAAAAC  CCGA
TAGGACTTGC  CATAGTTGTT  GATGTTTTTG  GGCTTCGA
      └─────────────────10──────────────────┘
                                          HindIII
```

The 134 base pair manufactured Fragment No. 1 illustrated in Table II having *XbaI* and *HindIII* sticky ends was inserted into an *E. coli* pBR322-derived cloning vector, pInt-γ-txB4, which contains a tryptophan synthetase (*trp*) promoter sequence and an *XbaI* site 3' to a Shine Dalgarno sequence followed by a structural gene for another polypeptide. See, co-owned, co-pending U.S. Patent Application Serial No. 483,451, by Alton, et al., filed April 15, 1983 [PCT International Publication No. WO83/04053, published November 24, 1983] and entitled "The Manufacture and Expression of Large Structural Genes", which is incorporated by reference herein. Plastmid pInt-γ-txB4 was digested with *XbaI* and *HindIII* endonucleases and after ligation of Fragment No. 1 into the vector the resulting plasmid, pInt-IL—2/1, was transformed for amplification into *E. coli* strain HB101. Clones with the fragment were characterized by agarose gel electrophoresis to verify the estimated molecular weight for the fragment.

To further characterize the cloned manufactured DNA, the base pair fragment was excised from plasmid Pint-IL—2/1 and inserted into single-strand bacteriophage M13mp10 and M13mp11 relicative form DNA at their *HindIII* and *XbaI* sites. Single-strand phages for both orientations were isolated and the DNA sequence for the IL—II Fragment No. 1 was verified using the Sanger Dideoxy sequencing technique.

Fragment No. 2 of a structural gene for human IL—II was assembled in a like manner from 24 oligonucleotides (numbered 11 through 34) and the sequence of this Fragment is set out in Table III.

## TABLE III

### IF-II FRAGMENT NO. 2

HindIII

```
                        ┌──────────11──────┐
                        AGCTTA CCCGTATGCT
                            AT GGGCATACGA
                        └──────────12──────┘


────────────────────────13────────────────────────────15───
GACTTTCAAA TTCTACATGC CGAAGAAAGC AACCGAACTG AAACACCTGC
CTGAAAGTTT AAGATGTACG GCTTCTTTCG TTGGCTTGAC TTTGTGGACG
──────────────────────14─────────────────────


────────────────────────17────────────────────────────
AGTGTCTGGA AGAAGAACTG AAACCTCTGG AGGAAGTTTT AAACCTGGCT
TCACAGACCT TCTTCTTGAC TTTGGAGACC TCCTTCAAAA TTTGGACCGA
──────16──────────────────────18──────────


──────19──────────────────────────────21──────────────
CAATCCAAGA ACTTTCATCT GCGTCCACGT GATCTGATCA GCAACATTAA
GTTAGGTTCT TGAAAGTAGA CGCAGGTGCA CTAGACTAGT CGTTGTAATT
──────20──────────────────────────22──────────


────23──────────────────────────────25──────────────
CGTTATCGTA CTGGAACTTA AAGGCTCTGA AACTACCTTC ATGTGCGAAT
GCAATAGCAT GACCTTGAAT TTCCGAGACT TTGATGGAAG TACACGCTTA
──────────────────24──────────────────26──────────


──────────27──────────────────────────29──────────────
ATGCAGACGA GACCGCTACC ATCGTGGAAT TTCTGAATCG TTGGATCACT
TACGTCTGCT CTGGCGATGG TAGCACCTTA AAGACTTAGC AACCTAGTGA
──────────────────28──────────────────30──────


──────────────31──────────────────────33──────────┐
TTCTGTCAGT CCATGATCAG CACTCTGACC TAATAGGATC CTAATAG
AAGACAGTCA GGTACTAGTC GTGAGACTGG ATTATCCTAG GATTATCAGCT
──────────────────32──────────────────34──────────┘
```

SalI

The 314 base pair manufactured Fragment No. 2, having *HindIII* and *SalI* sticky ends, was linked to Fragment No. 1, by insertion into plasmid pCFM414 (ATCC 40076; see Example 3), thereby creating plasmid 414 IL—2/3, according to procedures described in detail in Example 3 below. Clones containing the Fragment No. 2 were characterized by agarose gel electrophoresis to verify the estimated molecular weight for the fragment. To further characterize the cloned manufactured DNA, the fragment was excised from the plasmid 414 IL-2/3 and inserted into single-strand bacteriophage M13mp10 and M13mp11 relicative form DNA at their *HindIII* and *BamHI* sites. Clones with the inserted DNA in a defined orientation were isolated and characterized by polyacrylamide gel electrophoresis. Single-strand phages for both orientations were isolated and the DNA sequence for the IL—2 Fragment No. 2 was verified using the Sanger Dideoxy sequencing technique.

The assemblage of the two manufactured expression vector fragments in p414 IL—2/3 is set out in Table IV below.

## TABLE IV

```
                                    -1   1                                           10
                                    Met  Ala Pro Thr Ser Ser Ser Thr Lys Lys Thr
        ┌─────────────1─────────────┐ ┌────────────3────────────┐ ┌────5──────
5'- CTAGAAAAAA CCATGAGGGT AATAAATA ATG GCT CCT ACG AGC TCT TCT ACT AAG AAA ACC
3'-     TTTTTT GGTACTCCCA TTATTTAT TAC CGA GGA TGC TCG AGA AGA TGA TTC TTT TGG
     └──────────2─────────────┘     └──────────4──────────┘         └──────
     XbaI                                            SacI


                                    20                                          30
     Gln Leu Gln Leu Glu His Leu Leu Leu Asp Leu Gln Met Ile Leu Asn Gly Ile Asn Asn
     ─────────────────────────────────────7────────────┐ ┌──────────9───────
     CAG CTG CAA CTG GAA CAT CTG CTT CTT GAC CTG CAA ATG ATC CTG AAC GGT ATC AAC AAC
     GTC GAC GTT GAC CTT GTA GAC GAA GAA CTG GAC GTT TAC TAG GAC TTG CCA TAG TTG TTG
      ──────6──────────────────────────┘ └─────────8──────────┘


                                    40                                          50
     Tyr Lys Asn Pro Lys Leu Thr Arg Met Leu Thr Phe Lys Phe Tyr Met Pro Lys Lys Ala
     ──────────────────────11──────────┐ ┌────────────────────────────13───────
     TAC AAA AAC CCG AAG CTT ACC CGT ATG CTG ACT TTC AAA TTC TAC ATG CCG AAG AAA GCA
     ATG TTT TTG GGC TTC GAA TGG GCA TAC GAC TGA AAG TTT AAG ATG TAC GGC TTC TTT CGT
      ──────10──────────┘          └─────────12──────────┘
                     HindIII


                                    60                                          70
     Thr Glu Leu Lys His Leu Gln Cys Leu Glu Glu Glu Leu Lys Pro Leu Glu Glu Val Leu
     ───────────────────15──────────┐ ┌───────────────────17───────
     ACC GAA CTG AAA CAC CTG CAG TGT CTG GAA GAA GAA CTG AAA CCT CTG GAG GAA GTT TTA
     TGG CTT GAC TTT GTG GAC GTC ACA GAC CTT CTT CTT GAC TTT GGA GAC CTC CTT CAA AAT
     -14───────────────────┘ └─────────16──────────┘              └─────────18───────
                     PstI
```

0 136 489

TABLE IV (cont'd.)

```
                                              80                                              90
Asn Leu Ala Gln Ser Lys Asn Phe His Leu Arg Pro Arg Asp Leu Ile Ser Asn Ile Asn
─────────────────────19─────────────────           ───────────21──────────────
AAC CTG GCT CAA TCC AAG AAC TTT CAT CTG CGT CCA CGT GAT CTG ATC AGC AAC ATT AAC
TTG GAC CGA GTT AGG TTC TTC AAA GTA GAC GCA GGT GCA CTA GAC TAG TCG TCG TAA TTG
──────────────────────────20──────────           ───────────────22───────────
                                                                      BclI

                                              100                                            110
Val Ile Val Leu Glu Leu Lys Gly Ser Glu Thr Thr Phe Met Cys Glu Tyr Ala Asp Glu
───────────23──────────           ───────────25──────────────
GTT ATC GTA CTG GAA CTT AAA GGC TCT GAA ACT ACC TTC ATG TGC GAA TAT GCA GAC GAG
CAA TAG CAT GAC CTT GAA TTT CCG AGA CTT TGA TGG AAG TAC ACG CTT ATA CGT CTG CTC
──────────────24──────────           ──────────────26──────────

                                              120                                            130
Thr Ala Thr Ile Val Glu Phe Leu Asn Arg Trp Ile Thr Phe Cys Gln Ser Ile Ile Ser
─────27─────────           ──────────29──────────           ──────────31────────
ACC GCT ACC ATC GTG GAA TTT CTG AAT CGT TGG ATC ACT TTC TGT CAG TCC ATC ATC AGC
TGG CGA TGG TAG CAC CTT AAA GAC TTA GCA ACC TAG TGA AAG ACA GTC AGG TAG TAG TCG
────────────28──────────           ──────────────30──────────
      133
Thr Leu Thr

────────────────33────────────────
ACT CTG ACC TAA TAGGATC CTAATAG   3'
TGA GAC TGG ATT ATCCTAG GATTATCAGCT   5'
──32─────────────────34────────────

          BamHI          SalI
```

Included in Table IV is the numbered sequence of amino acid residues specified when the DNA sequence is suitably inserted in the proper reading frame within a microbial expression vector. The polypeptide sequence is thus seen to comprise the series of one hundred and thirty-three amino acids of naturally-occurring human IL—II together with an initial methionine residue (at position —1).

In the Table, codons specifying each amino acid are aligned below the designation of the residue specified. Bracketed regions in the DNA sequence indicate the thirty-four separate oligonucleotides initially formed and also designate the intermediate duplexes (e.g., 1 and 2, 3 and 4, etc.). Wherever consistent with the procedures for construction of the manufactured gene, coden usage is selected on the basis of "preferences" of the projected expression host (e.g., E. coli) according to the data set out in Grantham, et al., *Nucleic Acids Research, 8:* r49—r62 (1980); Grantham, et al., *Nucleic Acids Research, 8:* 1893—1912 (1980); and Grantham, et al., *Nucleic Acids Research, 9:* r43—r74 (1981). See also, Bennetzen, et al., *J. Biol. Chem., 257:* 3026—3031 (1982) and Grosjean, et al., *Gene, 18:* 199—209 (1982).

In the region of the manufactured gene 5' to the coden specifying Met$^{-1}$, there is provided a series of 20 base pairs,

$$5'—AA\ CCATGAGGGT\ AATAAATA—3'$$

$$3'—TT\ GGTACTCCCA\ TTATTTAT—5'$$

which comprise a duplicate of a ribosome binding site region of a highly expressed E. coli gene, outer membrane protein-F ("OMP—F") as described in U.S. Patent Application Serial No. 483,451 [PCT International Publication No. WO83/04053] by Alton, et al., *supra.* In the region 5' to the above-noted synthetic ribosome binding site sequence, base sequences are provided which establish an *Xbal* sticky end to facilitate insertion into vectors for amplification and expression. In the region 3' to the codon for the human IL—II carboxy terminal threonine residue, there are provided translation stop codons and sequences of bases generating an entire *BamHI* recognition site followed by a *Sall* sticky end.

Of interest in the construction revealed by Table IV are the multiple restriction endonuclease recognition sites which are unique to the manufacture sequence and, depending on the expression vector selected, would be unique to the vector after insertion of the manufactured sequence. The *HindIII* site is, of course, restored upon joining of the sticky ends of Fragment Nos. 1 and 2. Additionally, unique *Sacl, Pstl,* and *Bcll* sites are provided within the protein coding region of the manufactured gene without disturbing designation of the natural sequence of amino acid residues of human IL—II. Such internal unique restriction sites greatly facilitate construction of IL—II analogs according to the invention, such as are the subject of Example 5 below.

The following example relates to preparation of a DNA expression vector for use in transformation of selected microbial host cells to secure expression of polypeptide products of the invention.

## Example 3
### Construction of Plasmid 414 IL—2/3

To generate an expression vector which would provide for high levels of E. coli expression of human IL—II protein, a plasmid was constructed which placed the IL—II structural gene illustrated in Table IV under control of an E. coli tryptophan synthetase gene promoter. These manipulations involved use of plasmid pCFM414 which is the subject of co-owned, co-pending U.S. Patent Application Serial No. 521,964, by Morris, entitled "DNA Vector", filed August 10, 1983 concurrently with the parent application Serial No. 521,967 of the present invention. The disclosures of said application are specifically incorporated by reference herein. pCMF414 was deposited at the American Type Culture Collection, 12301 Parklawn Drive, Rockville, Maryland 20852, on July 22, 1983 in accordance with the Patent and Trademark Office's requirements for microorganism deposits, and was assigned number ATCC 40076. Briefly noted, plasmid pCFM414 includes a temperature-sensitive mutation in the copy control region. Upon transformation with this vector, host cell cultures grown at 30°C will have a low number of copies of the plasmid. The plasmid copy number increases fifty-fold or more (i.e., "runs away") within the host cells upon elevation of the culture temperature above 34°C. Growth at 37°C will ordinarily be lethal to the cells but prior to cell death there is an opportunity for multiple transcriptions of plasmid DNA.

The specific manipulations involved in construction of a vector using A.T.C.C. 40076 were as follows. Plasmid Pint-IL—2/1 (Example 2) was digested with restriction endonucleases *EcoRI* and *HindIII,* thereby excising a fragment containing a *trp* promoter originally present in Pint-γ-txB4 followed by the 134 base pair *Xbal-HindIII* sequence of Fragment No. 1 of IL—II.

Plasmid pCFM414 was digested with *EcoRI* and *Xhol* and the large fragment isolated. The 314 base pair manufactured IL—II Fragment No. 2 (Table III) having *HindIII* and *Sall* sticky ends and the *EcoRI-HindIII* fragment containing IL—II Fragment No. 1 in association with a *trp* promoter purified from Pint-IL—2/1 were jointly inserted into the large fragment of plasmid pCFM414, to generate plasmid 414 IL—2/3. Plasmid 414 IL—2/3 thereby contained the complete structural gene appearing in Table IV in association with a functional *trp* promoter.

This construction retained an intact *EcoRI* recognition site and while ligation of the complementary *Sall* and *Xhol* sticky ends did not restore either recognition site, *BamHI* recognition sites are present on both sides of the juncture. The IL—II gene and the preceding *trp* promoter may thus be removed by digestion with *EcoRI* and *BamHI,* while the gene alone may be removed using *XbaI* and *BamHI.* It is noteworthy that this construction resulted in the placement of the IL—II structural gene 5' to lambda phage-derived transcription terminator sequence, "Toop", adjacent the *XhoI* and *BamHI* sites in pCFM414.

A functional equivalent of plasmid 414 IL—2/3 lacking twelve non-essential base pairs could readily be constructed with IL—II Fragment Nos. 1 and 2 by ligating the Fragments together, digesting the product with *BamHI* (to delete the bases 3' to the *BamHI* site in Fragment No. 2) and thereafter inserting the gene as an *XbaI* to *BamHI* fragment into the large fragment resulting from *XbaI* and *BamHI* digestion of plasmid pADH59. Plasmid pADH59 was deposited at the American Type Culture Collection, 12301 Parklawn Drive, Rockville, Maryland 20852, on April 14, 1983, under number ATCC 39335, in accordance with the Patent and Trademark Office's requirements for microorganism deposit and was converted to the deposit requirements of the Budapest Treaty on December 20, 1983. As described in co-owned, co-pending U.S. Patent Application Serial No. 486,091 filed April 25, 1983 by Banks, et al. which is incorporated by reference herein, plasmid pADH59 includes a manufactured structural gene coding for human urogastrone.

As was the case with the IL—II Fragment No. 1 in the construction of intermediate plasmid pInt-IL—2 above, the entire urogastrone gene was first inserted following the *trp* promoter/regulator into plasmid pInt-γ-txB4 (digested with *XbaI* and the *SalI* rather than *HindIII*). The urogastrone gene and the preceding *trp* promoter/regulator were then excised from the intermediate plasmid with *EcoRI* and *SalI* and inserted into plasmid pCFM414 which had been digested with *EcoRI* and *XhoI.* While the *SalI* recognition site was not restored in this construction, a *BamHI* site immediately 3' to the *XhoI* site in pCFM414 was undisturbed in pADH59. Digestion of pADH59 with *XbaI* and *BamHI* and insertion of the *BamHI* digested product of ligation of IL—II Fragment Nos. 1 and 2 therefore generates a plasmid which duplicates plasmid 414 IL—2/3 except for twelve non-essential base pairs between the two adjacent *BamHI* sites therein.

The following example relates to microbial expression of IL—II.

## Example 4

*E. coli* JM103 transformed with plasmid 414 IL—2/3 is grown in TE (1% Tryptone, 0.5% Yeast Extract, and 0.5% NaCl) containing 100 µg/ml Ampicillin at 30°C. When the cultures reach an optical density of 0.5 $A_{600}$ then they are shifted to 37°C. The cultures are harvested by centrifugation approximately 3—4 hours after the temperature shift. The cells are resuspended in ice cold 20mM Tris pH 7.5 and 50 mM NaCl at a concentration of 6—7 mls per OD-liter. The cells are then lysed by passage through a Gaulin homogenizer three times at 6000 psi (0.87 Pa). The lysed cells are centriuged at 8500 in JA10 for 25 minutes. The pellet is resuspended in 2 ml $H_2O$ per OD-liter of cells and then respun as before. The pellet is then resuspended again at the same concentration in water and 1M Tris, pH7.5 added to bring the final Tris concentration to 10mM. Then 20% sodium dodecyl sulfate (SDS) is added to bring the final concentration of SDS to 1%. The sample is gently shaken at room temperature (RT) for 1 hour then spun at 17,000 in a JA20 rotor for 25 minutes. Analysis of whole cell extracts by SDS—PAGE reveal the presence of an appropriately sized protein (MW about 15,000 daltons). The crude supernate can then be diluted serially 10 fold in RPMI 1640 containing 10% fetal calf serum. Samples diluted to $10^{-3}$—$10^{-6}$ can be run in the $^3$H thymidine incorporation assay. Samples diluted only $10^{-1}$—$10^{-2}$ still contain too much SDS to allow cell viability. The $^3$H-thymidine incorporation assay measures the incorporation of the label into nucleic acid. [Gillis, et al., *J. Immunol., 120:* 2027—2032 (1978); and Oppenheim, et al., p. 81, *Manual of Clinical Immunology,* American Society for Microbiology, N. Rose and H. Friedman, eds. (1976)]. Two week old human peripheral blood lymphocyte (PBL) cultures stimulated with PHA and maintained with exogenously added IL—II become dependent on IL—II for growth [Mier, et al., *PNAS (USA), 77:* 6134—6138 (1980); and Gillis, et al., *supra*]. A continuous murine tumor-specific cytotoxic T cell line, CTL L2 is also highly dependent on the addition of IL—II to support growth. In order for $^3$H-thymidine to be incorporated into nucleic acid in either 14 day old human PBL or Mouse CTL L2 cells, they must be actively growing. If one removes the source of IL—II from either cell culture, the cells will stop growing and ultimately die within a day or two. Thus one can use the $^3$H-thymidine incorporation assay to determine whether the IL—II produced by plasmid 414IL—2/3 *E. coli* is active. The bacterially synthesized IL—II does, in fact, maintain the growth of IL—II dependent cells as determined by the $^3$H-thymidine incorporation assay.

The following examples relates to the manufacture of polypeptide analogs of human IL—II.,

## Example 5

One class of human IL—II analogs include those wherein one of the three cysteine residues of the naturally-occurring form of the polypeptide is replaced by another amino acid residue, such as serine or alanine. Three such analogs have been manufactured and designated [Ser[58]]IL—II, [Ser[105]]IL—II, and [Ser[125]]IL—II.

The above-mentioned analogs were prepared from the manufactured sequence of Table IV by altering the region spanning the *HindIII* to *BamHI* sites, and employing the following oligonucleotides in Table V below which were manufactured according to Example 1. The single base change is underscored in each oligonucleotide.

## TABLE V

| Analog | Manufactured Oligonucleotide |
|---|---|
| [Ser$^{58}$]IL-II | CACCTGCAGTCTCTGGAAGAA |
| [Ser$^{105}$]IL-II | ACCTTCATGTCCGAATATGCAG |
| [Ser$^{125}$]IL-II | GATCACTTTCTCTCAGTCCATCA |

Each oligonucleotide was then employed in the site-directed mutagenesis procedure according to Gillam, et al., *Gene, 12:* 129—137 (1980), with the following changes:

Only 0.5 pmole of primer (kinases) was used to give a 10:1 (rather than a 30:1) molar excess of primer to template. The primer-template mixture was cooled to room temperature from 65°C slowly over 10 minutes. *E. coli* JM103 was used for translation using dilutions of the DNA mixture. One unit of Klenow (Boehringer-Mannheim) was used in a 4—5 hour reaction. Hybridizations were done for two hours with 50,000,000 cpm probe and the high temperature wash was done for 15 minutes immediately after the room temperature washes without setting an exposure on film. The wash temperatures used were 63°C for all three Cys→Ser analogs.

After sequencing, each analog was treated with the enzyme RNAse and then was phenol extracted and ETOH precipitated. The DNA was cut with *HindIII* and *BamHI* and run on 1.2% agarose gel. Thereafter, the IL—2 *HindIII—BamHI* fragments were isolated by NA45 paper and used in a ligation with 414 IL—2/3 DNA (see Example 3) cut with *HindIII* and *BamHI* and NA45 gel purified. The transformation was done in JM103 and 20—30 fold increases over background (no insert) were obtained.

Colonies were picked and grown at 30°C, then shifted to 37°C at an OD of approximately 0.2. After 3 hours, samples were taken for gels (0.5 ODml); for assay (1 ODml was centrifuged and resuspended in 60 μl 1% SDS; DTT was added to half of some samples); and for minipreps.

Such analogs were expected to retain the biological activity of the natural form but be substantially easier to recover in biologically active form from microbial expression systems owing to a decreased likelihood of improper intramolecular folding or intermolecular dimer or polymer formation. Only the [Ser$^{125}$]IL—II analog maintained full activity in assays. [Ser$^{105}$]IL—II and [Ser$^{58}$]IL—II were one hundred fold less active than the naturally occurring sequence.

The following example is directed to changes in the design of the Shine Delgarno sequences preceding the IL—II coding sequences.

### Example 6

The IL—II gene illustrated in Table IV, *supra,* contains the following sequence of nucleotides preceding the initiation coden — — AGT — —:

5'—CAT GAG GGT AAT AAA TA—3'

The first nine bases therein comprise the Shine Delgarno sequence, or ribosome binding site; the remainder of the sequence functions as a spacer between the ribosome binding site and the coding sequence.

In order to study the effect of Shine Delgarno sequences on the expression levels of the IL—II gene, the following three ribosome binding sites were designed and synthesized:

| | Shine Delgarno | spacer |
|---|---|---|
| (a) | CAA GAG GGT | AAT AAA TA |
| (b) | CAT GGA GGT | AAT AAA TA |
| (c) | CAA GGA GGT | AAT AAA TA |

To prepare these three new ribosome binding sites, the following segments were synthesized according to the procedures of Example 1, *supra,* to replace segments 1 and 2 in Table IV.

| Segment Number | |
|---|---|
| 1a | 5'-CTA GAA AAA ACC AAG AGG GTA ATA AAT-3' |
| 2a | 5'-CCA TTA TTT ATT ACC CTC TTG GTT TTT-3' |
| 1b | 5'-CTA GAA AAA ACC ATG GAG GTA ATA AAT-3' |
| 2b | 5'-CCA TTA TTT ATT ACC TCC ATG GTT TTT-3' |
| 1c | 5'-CTA GAA AAA ACC AAG GAG GTA ATA AAT-3' |
| 2c | 5'-CCA TTA TTT ATT ACC TCC TTG GTT TTT-3' |

These segments were annealed and ligated to segments 3 and 4 as in Example 2, *supra,* of Table IV, and then cut with the endonuclease enzyme *SacI* to provide new *XbaI—SacI* fragments to replace the original fragment appearing in Table IV.

A new vector containing sequence c was constructed as described in the above examples, and resulted in a two-fold increase in human IL—II production.

The foregoing illustrative examples are principally directed to construction of a manufactured IL—II gene suited for direct expression in *E. coli* host cells of a [Met$^{-1}$]IL—II product. The manufactured gene could as readily be constructed in a manner especially well-suited for direct expression in yeast cells through use of yeast preference codons. Further, the procedures of co-owned, co-pending U.S. Patent Application Serial No. 487,753, filed April 22, 1983 by Bitter and entitled, "Secretion of Exogenous Polypeptides From Yeast" could be employed to secure yeast expression coupled with secretion of the polypeptide into the growth medium. In such a case it would be unnecessary to provide a methionine-specifying, initiating, ATG codon 5' to the IL—II polypeptide specifying codons.

Products of the present invention and/or antibodies thereto may be suitably "tagged", for example radiolabelled (e.g., with I$^{125}$) conjugated with enzymes or fluorescently labelled, to provide reagent materials useful in assays and/or diagnostic test kits, for the qualitative and/or quantitative determination of the presence of such products and/or said antibodies in fluid samples. Such antibodies may be obtained from the inoculation of one or more animal species (e.g., mice, rabbit, goat, human, etc.) or from monoclonal antibody sources. Any of such reagent materials may be used alone or in combination with a suitable substrate, e.g., coated on a glass or plastic particle or bead.

## Claims

1. A polypeptide analog of human Interleukin II in which the cysteine residue at amino acid position 125, numbered in accordance to the naturally occurring human Interleukin II, is replaced by another amino acid residue and which substantially retains the biological activity of the natural form.

2. An analog according to Claim 1, wherein the Cys$^{125}$ is replaced by Ser$^{125}$ or Ala$^{125}$.

3. An analog according to Claim 1 or 2, which comprises the [Met$^{-1}$] form thereof

4. A polypeptide analog according to claim 2 which is human (Ser$^{125}$) Interleukin II.

5. A polypeptide analog according to claim 2 which is human (Ala$^{125}$) Interleukin II.

6. A polypeptide analog comprising the [Met$^{-1}$] form of the analog of Claim 4 or 5.

7. A composition containing a polypeptide analog in accordance with any one of the preceding claims.

8. A composition according to Claim 7, which comprises the polypeptide analog suitably "tagged", e.g. radio-labelled or conjugated with enzyne or fluorescently labelled to provide a reagent material for use in an assay and/or diagnostic kit or for qualitative and/or quantitative determination of the presence of said polypeptide.

9. A process of preparing a polypeptide analog of human Interleukin II according to claim 1 which process comprises:

a) manufacturing a synthetic gene capable of directing the synthesis in a selected host microorganism of a human Interleukin II polypeptide;

b) altering in said gene the codon specifying the cysteine residue in position 125, numbered in accordance to the naturally occurring human Interleukin II polypeptide, so as to replace it with a codon specifying another aminoacid residue;

c) transforming a selected host microorganism with a biologically functional DNA including said altered synthetic gene of b) and growing, under appropriate nutrient conditions, said transformed microorganism, whereby said microorganism expresses said gene and produces the polypeptide analog.

10. A process according to Claim 9, wherein the codon specifying Cys$^{125}$ is replaced by a codon specifying either Ser$^{125}$ or Ala$^{125}$.

11. A process according to Claim 9 or 10, which comprises making the [Met$^{-1}$] form of the analog.

## Patentansprüche

1. Polypeptidvariante des menschlichen Interleukins-II, dadurch gekennzeichnet, daß der Cysteinrest an der Aminosäureposition 125, nummeriert in Übereinstimmung mit dem natürlich auftretenden menschlichen Interleukin-II, durch einen anderen Aminosäurerest ersetzt ist und daß sie im wesentlich die biologische Aktivität der natürlichen Form beibehält.

2. Variante nach Anspruch 1, dadurch gekennzeichnet, daß das Cys$^{125}$ durch Ser$^{125}$ oder Als$^{125}$ ersetzt ist.

3. Variante nach Anspruch 1 oder 2, dadurch gekennzeichnet, daß sie die [Met$^{-1}$]-Form davon umfaßt.

4. Polypeptidvariante nach Anspruch 2, dadurch gekennzeichnet, daß sie menschliches (Ser$^{125}$)-Interleukin-II ist.

5. Polypeptidvariante nach Anspruch 2, dadurch gekennzeichnet, daß sie menschliches (Ala$^{125}$)-Interleukin-II ist.

6. Polypeptidvariante, dadurch gekennzeichnet, daß sie die [Met$^{-1}$]-Form der Variante von Anspruch 4 oder 5 umfaßt.

7. Zusammensetzung, gekennzeichnet durch einen Gehalt an einer Polypeptidvariante nach einem der vorangehenden Ansprüche.

8. Zusammensetzung nach Anspruch 7, dadurch gekennzeichnet, daß sie die in geeigneter Weise markierte, z.B. isotopenmarkierte oder mit Enzym konjugierte oder fluoreszierend markierte, Polypeptidvariante umfaß, um einen Nachweisstoff zur Verwendung in einem Analyse- und/oder Diagnosesatz oder für die qualitative und/oder quantitative Bestimmung des Vorhandenseins von besagtem Polypeptide zur Verfügung zu stellen.

9. Verfahren zum Herstellen einer Polypeptidvariante des menschlichen Interleukins-II nach Anspruch 1, dadurch gekennzeichnet, daß das Verfahren folgende Schritte umfaßt:

a) Herstellen eines synthetischen Gens, das in der Lage ist, in einem ausgewählten Wirtsmikroorganismus die Synthese eines menschlichen Interleukin-II Polypeptids zu steuern;

b) Ändern desjenigen Codons in besagtem Gen, welches den Cysteinrest in Position 125, nummeriert in Übereinstimmung mit dem natürlich auftretenden menschlichen Interleukin-II-Polypeptid, spezifiziert, um es durch ein Codon zu ersetzen, das einen anderen Aminosäurest spezifiziert;

c) Umformen eines ausgewählten Wirtsmikroorganismusses mit einer biologisch funtionellen DNA, die besagtes geändertes synthetisches Gen aus b) enthält, und Züchten besagten umgeformten Mikroorganismusses unter geeigneten Nährstoffbedingungen, wodurch besagter Mikroorganismus besagtes Gen exprimiert und die Polypeptidvariante produziert.

10. Verfahren nach Anspruch 9, dadurch gekennzeichnet, daß dasgenige Codon, das Cys$^{125}$ spezifiziert, durch ein Codon ersetzt wird, das entwẹder Ser$^{125}$ oder Als$^{125}$ spezifiziert.

11. Verfahren nach Anspurch 9 oder 10, dadurch gekennzeichnet, daß es das Herstellen der [Met$^{-1}$]-Form der Variante umfaßt.

**Revendications**

1. Analogue polypeptidique de l'Interleukin II humaine dans lequel le résidu de cystéine à la position d'acide aminé 125, numérotée conformément à l'Interleukin II humaine se produisant naturellement, est remplacé par un autre résidu d'acide aminé, et qui conserve sensiblement l'activité biologique de la forme naturelle.

2. Analogue selon la revendication 1, dans lequel la Cys$^{125}$ est remplacée par le Ser$^{125}$ ou l'Ala$^{125}$.

3. Analogue selon la revendication 1 ou 2, qui comprend sa forme [Met$^{-1}$].

4. Analogue polypeptidique selon la revendication 2 qui est de l'Interleukin II humaine (Ser$^{125}$).

5. Analogue polypeptidique selon la revendication 2 qui est de l'Interleukin II humaine (Ala$^{125}$).

6. Analogue polypeptidique comprenant la forme [Met$^{-1}$] de l'analogue de la revendication 4 ou 5.

7. Composition contenant un analogue polypeptidique selon l'une quelconque des revendications précédentes.

8. Composition selon la revendication 7, qui comprend l'analogue polypeptidique convenablement "marqué", par exemple radiomarqué ou conjugué avec une enzyme ou marqué par fluorescence, pour fournir un réactif utilisable dans un nécessaire d'essai et/ou de diagnostic ou pour la détermination qualitative et/ou quantitative de la présence dudit polypeptide.

9. Procédé de préparation d'un analogue polypeptidique de l'Interleukin II humaine selon la revendication 1, lequel procédé comprend:

a) fabriquer un gène synthétique capable de diriger la synthèse dans un micro-organisme hôte d'un polypeptide de l'Interleukin II humaine;

b) altérer dans ledit gène le codon désignant le résidu de cystéine en position 125, numérotée conformément au polypeptide d'Interleukin II humaine se produisant naturellement, de façon à le remplacer par un codon désignant un autre résidu d'acide aminé;

c) transformer un micro-organisme hôte choisi avec un DNA biologiquement fonctionnel incluant ledit gène synthétique altéré de b), et cultiver, dans es conditions nutritives appropriées, ledit micro-organisme transformé, ledit micro-organisme exprimant ledit gène et produisant l'analogue polypeptidique.

10. Procédé selon la revendication 9, dans lequel le codon désignant la Cys$^{125}$ est remplacé par un coden désignant soit la Ser$^{125}$, soit l'Ala$^{125}$.

11. Procédé selon la revendication 9 ou 10, qui comprend la réalisation de la forme [Met$^{-1}$] de l'analogue.